(19) Europäisches Patentamt
European Patent Office
Office européen des brevets

(11) **EP 3 957 239 B1**

(12) **EUROPEAN PATENT SPECIFICATION**

(45) Date of publication and mention
of the grant of the patent:
**06.11.2024 Bulletin 2024/45**

(21) Application number: **19924783.4**

(22) Date of filing: **18.04.2019**

(51) International Patent Classification (IPC):
***A61B 5/0531*** (2021.01)   ***A61B 5/11*** (2006.01)

(52) Cooperative Patent Classification (CPC):
**A61B 5/0531; A61B 5/1126**

(86) International application number:
**PCT/CN2019/083290**

(87) International publication number:
**WO 2020/211051 (22.10.2020 Gazette 2020/43)**

(54) **NON-INVASIVE METHOD AND SYSTEM FOR MEASURING MOTION FEATURE OF MYOCARDIAL TISSUE**

NICHTINVASIVES VERFAHREN UND SYSTEM ZUR MESSUNG EINES BEWEGUNGSMERKMALS VON HERZMUSKELGEWEBE

PROCÉDÉ ET SYSTÈME NON INVASIFS POUR MESURER UNE CARACTÉRISTIQUE DE MOUVEMENT D'UN TISSU MYOCARDIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.02.2022 Bulletin 2022/08**

(73) Proprietor: **Msheaf Health Management Technologies Limited**
**Kowloon, Hong Kong 999077 (CN)**

(72) Inventors:
• **WANG, Ling**
**Hong Kong 999077 (CN)**
• **YI, Cheng**
**Hong Kong 999077 (CN)**

• **HE, Bixia**
**Hong Kong 999077 (CN)**
• **XIE, Peng**
**Hong Kong 999077 (CN)**

(74) Representative: **Winter, Brandl - Partnerschaft mbB**
**Alois-Steinecker-Straße 22**
**85354 Freising (DE)**

(56) References cited:
**EP-A1- 3 957 240    WO-A1-98/53737**
**CN-A- 101 022 765    CN-A- 108 922 580**
**US-A1- 2005 283 091    US-A1- 2010 225 303**
**US-B1- 6 339 722**

## Description

## TECHNICAL FIELD

The present invention relates to a technology for measurement of biological tissues, in particular to a non-invasive method and system for measuring the motion characteristics of a myocardial tissue.

## BACKGROUND

The basic function of a heart is to pump blood, circulate the blood in an organism, and provide oxygen and nutrients to tissues. Therefore, the measurement of cardiac dynamics parameters is of extremely important significance in the medical field. Myocardial cells have structural characteristics indicating that they pertain to an elastic tissue. Therefore, the motion of the myocardial tissue, especially elasticity thereof, should be the main measurement target. At present, the stress-strain relationship of the myocardial tissue has been extensively studied, and its related applications are mainly realized through ultrasonic imaging systems.

A heart has four chambers, including two atria and two ventricles. Under normal circumstances, the right atrium collects blood from the superior and inferior vena cava. The blood then enters the right ventricle, where the blood is pumped into the lungs. The left atrium receives blood from the pulmonary veins and sends it to the left ventricle, which pumps the blood through the aorta to the whole body. The heart wall has a three-layer structure, namely the inner endocardium, the middle myocardium and the outer epicardium. The endocardium is the lining of simple squamous epithelium, covering the heart chambers and valves. The myocardium is the muscle of the heart, a layer of involuntary striated muscle tissue, which is restricted by the framework of collagen, so that the myocardial cells are arranged on a curved sheet, forming a spiral structure as a whole. The myocardium is the focus of the present invention. The pericardium is a double-layered sac containing the heart and the roots of large blood vessels.

Under pathological conditions, the two main situations of most concern are hypertension and myocardial ischemia. Long-term hypertension will eventually lead to ventricular hypertrophy and even heart failure. Ischemia, mainly caused by coronary artery stenosis, will eventually trigger heart attacks, followed by myocardial infarctions, and lead to a heart failure. The present invention focuses on the early detection of changes in the myocardial tissue and can be used to prevent sudden heart attacks.

There are many methods that can measure cardiac function at different levels, such as organic, tissue, and cellular levels. At the organic level, the estimation of ventricular volume can be done through image construction. Stroke volume (SV) and ejection fraction (EF) can also be measured, which represent the overall pumping function of the heart. But these parameters do not explain the mechanical properties of the tissues. Direct measurement of strain on the ventricular wall has been proved to be a very important measurement of activity of the myocardial tissue, which can indirectly reflect cardiac function. At present, such measurement is mainly done by ultrasonic Doppler or ultrasonic speckle technology on paired speckles. Contracted LV torsion from ultrasonic speckle tracking imaging is another technology for assessing cardiac function. At the same time, the omni-directional longitudinal strain has also been proved to be a useful tool for predicting cardiotoxicity in chemotherapy.

On the one hand, currently, there has been no non-invasive measurement method for the health state of the myocardial tissue at the cellular level. On the other hand, even though current technologies can diagnose some health states of the myocardial tissue, they have some disadvantages. For example, the MRI imaging method is very expensive. Although ultrasonic imaging is less expensive, it is still affected by many aspects. First, ultrasonic imaging cannot be performed continuously or for a long time. Second, the resolution of ultrasonic imaging results is not high, and the results of ultrasonic imaging also depend on patients. The imaging results will vary due to the lack of standardized operations, and the cost of ultrasonic imaging is still high.

Many researchers have conducted a large number of previous studies on the invasive characterizations of the myocardial tissue in animals and humans. Studies have shown that ischemia can lead to changes in the impedance of the myocardial tissue, which proves the changes in the impedance of the heart tissue during the cardiac cycle. All the results support the present invention.

In many measurements of cell parameters, cell size is in need of use for standardization. In equipotential cells, standardization relies on the cell surface area obtained from capacitance measurement. This is a widely used technology, of which the theoretical basis is that the membrane capacitance is directly proportional to the cell surface area. The membrane capacitance in this theory is different from the capacitance in the present invention. The former is the capacitance across the membrane, while the capacitance in the present invention is the capacitance from the membrane to the infinity or ground. Their physical and mathematical basis is that the capacitance in the present invention is proved to be directly proportional to the average longitudinal length of the cells. The present invention is the application of this principle in a system for measuring the motion characteristics of the myocardial tissue.

The related document WO 98/53737 A1 discloses a method and a system for non-invasively determining at least one main cardiorespiratory parameter of an individual, such as the Stroke Volume, at least one parameter characterizing balance of the extracellular fluid in the body (such as the Index Balance), and for diagnostics of blood circulatory problems and/or failures of cardiac functions.

Further, the document EP 3 957 240 A1 discusses about a non-invasive method and system to detect characteristic information of body tissues, comprising applying multiple synchronous alternating currents at different frequencies to a human body.

## SUMMARY OF THE INVENTION

In order to solve the problems in the prior art, the present invention proposes a non-invasive method for measuring the motion characteristics of a myocardial tissue, with the purpose of calculating the average longitudinal length of myocardial cells by measuring the overall capacitance of the heart tissue, thereby obtaining the motion characteristics of the myocardial tissue. The invention is set out in the appended claim set. The method is mainly used for detecting information for non-therapeutic purposes.

To achieve the above objective, the present invention provides a non-invasive method for measuring the motion characteristics of a myocardial tissue, wherein the method comprises: transmitting a plurality of generated synchronous orthogonal, phase controllable and adjustable alternating currents with different frequencies into an organism so as to generate a plurality of synchronous periodic alternating current (AC) voltage signals with different frequencies; receiving the periodic AC voltage signals modulated by changes in the organism's heart tissue to obtain the organism's frequency responses; calculating resistances and capacitances of the heart tissue according to the frequency responses; and estimating the motion characteristics of the myocardial tissue according to the resistances and the capacitances.

Preferably, the calculating resistances and capacitances of the heart tissue according to the frequency responses comprises, obtaining system transfer function of the organism according to the frequency responses, and performing multi-chamber modeling to separate the heart tissue and peripheral tissues.

The estimating the motion characteristics of the myocardial tissue according to the resistances and the capacitances comprises: calculating the average longitudinal length of myocardial cells and its change according to the capacitances, and/or calculating heart pumping blood flow according to the resistances; and obtaining the overall longitudinal elastic state of the heart according to the average longitudinal length of the myocardial cells and its change and/or the heart pumping blood flow.

Preferably, the method further comprises, estimating health and working states of the heart and the myocardium according to the overall longitudinal elastic state of the heart.

Preferably, the estimating comprises, analyzing the health and working states of the heart and the myocardium according to the slope value of changes of the overall longitudinal elastic state of the heart, the delay to an R wave, the peak-to-peak value, and the change curve and its derivative's shape of the average longitudinal length of the myocardial cells, wherein the health and working states of the heart and the myocardium comprise, the systole speed, time, intensity and pattern of the heart tissue, and/or the diastole speed, time, recovery and pattern of the heart tissue.

Preferably, the obtaining the organism's frequency responses comprises, calculating a frequency response estimation value of a specific frequency every 0.25 to 5 milliseconds.

Preferably, the calculating the average longitudinal length of myocardial cells and its change according to the capacitances comprises: detecting the average longitudinal length of the myocardial cells and its change over time at a rate of 200 to 4000 times per second; and processing the time sequence of the change over time of the average longitudinal length of the myocardial cells using a digital signal processing method, wherein the digital signal processing method comprises digital filtering, Fast Fourier Transform (FFT) , and time domain and frequency domain analysis.

Preferably, the method further comprises, referring to an electrocardiogram having the same time sequence to analyze the change sequence of the average longitudinal length of the myocardial cells, wherein the referring comprises comparing the electrocardiogram with the change sequence of the average longitudinal length of the myocardial cells for their cardiac cycles, systolic and diastolic phases, and/or the boundaries thereof.

Preferably, the performing multi-chamber modeling to separate the heart tissue and peripheral tissues comprises, modeling each chamber as parallel resistor and capacitor, multiple chambers being connected in series or in parallel.

In order to achieve the above objective, the present invention also provides a system for implementing the above methods, wherein the system comprises a terminal and at least one processor, wherein the terminal comprises a generator for transmitting a plurality of generated synchronous orthogonal, phase controllable and adjustable, and periodic alternating currents with different frequencies; and one or more sensors for transmitting the periodic alternating currents into an organism to generate a plurality of periodic AC voltage signals with different frequencies, and receiving the periodic AC voltage signals modulated by changes in the heart tissue of the organism to obtain the organism's frequency responses; wherein the processor is configured to calculate resistances and capacitances of the heart tissue according to the frequency responses, and to estimate the motion characteristics of the myocardial tissue according to the resistances and the capacitances, wherein the processor is configured to calculate an average longitudinal length of myocardial cells and its change according to the capacitances, and/or calculating heart pumping blood flow according to the resistances, and obtain the overall longitudinal elastic state of the heart according to the average longitudinal length of the myocardial cells and its change and/or the heart pumping blood flow.

Preferably, the sensor is configured to collect single or multiple pieces of data from different parts.

Preferably, the system may comprise a database for storing processing results and data of the processor, and the processor may retrieve the database.

Preferably, the processor may be remote, and may be used for remote observation of the system's work in a real-time mode.

Preferably, the terminal further comprises a man-machine interface for controlling the system and/or displaying results.

Compared with the prior art, the present invention relates to a new technology for detecting the contraction and relaxation of the myocardial tissue at the cellular level, and has the advantage that the present invention provides a continuousand non-invasive method with high-sampling rate to measure the motion of the myocardial tissue at an overall cellar level, so as to detect even more subtle abnormal changes in the myocardial cells. In addition, the present invention avoids the traditional technology of using imaging results for analysis, and provides a faster but standard measurement method with lower cost.

## BRIEF DESCRIPTION OF THE DRAWINGS

In order to illustrate the embodiments of the present invention or the technical solutions of the prior art more clearly, the following will briefly introduce the drawings that need to be used in the description of the embodiments or the prior art. Obviously, the drawings in the following description are merely some of the embodiments of the present invention, those of ordinary skill in the art also can obtain other drawings based on these drawings without creative work.

FIG. **1** is a schematic diagram of a two-dimensional abstract model of simulated myocardial cells provided by an embodiment of the present invention;

FIG. **2** is an overall frame diagram of a part of the system provided by another embodiment of the present invention;

FIG. **3** is a schematic diagram illustrating the placement of the transmitting and receiving electrodes provided by another embodiment of the present invention;

FIG. **4** is a schematic diagram of a circuit structure of the system provided by another embodiment of the present invention;

FIGS. **5**a-**5**d are flowcharts of a method provided by another embodiment of the present invention;

FIGS. **6**a-**6**d are schematic diagrams of a young man's electrocardiogram, curves of his heart's resistance and capacitance over time, and the derivative of the capacitance curve provided by another embodiment of the present invention;

FIGS. 7a-7d are schematic diagrams of a normal middle-aged man's electrocardiogram, curves of his heart's resistance and capacitance over time, and the derivative of the capacitance curve provided by another embodiment of the present invention;

FIGS. **8**a-**8**d are schematic diagrams of an elderly woman's electrocardiogram, curves of her heart's resistance and capacitance over time, and the derivative of the capacitance curve provided by another embodiment of the present invention;

FIGS. 9a-9d are schematic diagrams of an elderly woman's electrocardiogram, curves of her heart's resistance and capacitance over time, and the derivative of the capacitance curve provided by another embodiment of the present invention;

FIGS. 10a-10d are schematic diagrams of an elderly woman's electrocardiogram, curves of her heart's resistance and capacitance over time, and the derivative of the capacitance curve provided by another embodiment of the present invention;

FIGS. 11a-11d are schematic diagrams of an elderly woman's electrocardiogram, curves of her heart's resistance and capacitance over time, and the derivative of the capacitance curve provided by another embodiment of the present invention;

FIGS. 12a-12d are schematic diagrams of an elderly woman's electrocardiogram, curves of her heart's resistance and capacitance over time, and the derivative of the capacitance curve provided by another embodiment of the present invention;

FIGS. 13a-13d are schematic diagrams of a normal person's electrocardiogram, curves of his/her heart's resistance and capacitance over time, and his/her heart's average cell deformation rate (similar to tensor change rate) provided by another embodiment of the present invention;

FIGS. 14a-14d are schematic diagrams of a normal person's electrocardiogram, curves of his/her heart's resistance and capacitance over time, and his/her heart's average cell deformation rate (similar to tensor change rate) provided by another embodiment of the present invention;

FIGS. 15a-15d are schematic diagrams of a normal person's electrocardiogram, curves of his/her heart's resistance and capacitance over time, and his/her heart's average cell deformation rate (similar to tensor change rate) provided by another embodiment of the present invention;

FIGS. 16a-16d are schematic diagrams of a normal person's electrocardiogram, curves of his/her heart's resistance and capacitance over time, and his/her heart's average cell deformation rate (similar to tensor change rate) provided by another embodiment of the present invention;

FIGS. 17a-17d are schematic diagrams of the electrocardiogram of a person with an abnormal heart tissue, curves of his/her heart's resistance and capacitance over time, and his/her heart's average cell deformation rate (similar to tensor change rate) provided by another embodiment of the present invention;

FIGS. 18a-18d are schematic diagrams of the electrocardiogram of a person with an abnormal heart tissue, curves of his/her heart's resistance and capacitance over time, and his/her heart's average cell deformation rate (similar to tensor change rate) provided by another embodiment of the present invention;

FIGS. 19a-19d are schematic diagrams of the electrocardiogram of a person with an abnormal heart tissue, curves of his/her heart's resistance and capacitance over time, and his/her heart's average cell deformation rate (similar to tensor change rate) provided by another embodiment of the present invention;

FIGS. 20a-20d are schematic diagrams of the electrocardiogram of a person with an abnormal heart tissue, curves of his/her heart's resistance and capacitance over time, and his/her heart's average cell deformation rate (similar to tensor change rate) provided by another embodiment of the present invention.

## DETAILED DESCRIPTION OF EMBODIMENTS

The embodiments of the present invention will now be described in further detail with reference to the drawings. These drawings are all simplified schematic diagrams, which merely illustrate the basic structure of the present invention in a schematic manner, so they only show the constitution relevant to the present invention.

The present invention relates to a non-invasive technology for detecting the electrical properties of tissues in an organism, such as resistances and capacitances of the tissues and their change patterns. Its goal is to capture changes in body fluid, blood flow and cardiovascular circulatory tissues, to monitor the health state of the organism, to measure and verify the elasticity of the cardiovascular system, and to detect information for non-therapeutic purposes.

In the embodiments provided by the present invention, heart cells are considered to be equipotential. Therefore, the cell size can be estimated by capacitance measurement. When the heart cells are in their normal positions, it can be considered that they are arranged in series and parallel modes at the same time, because the heart's structural cells restrict the muscle cells in space. Assuming that normal human heart cells have similar volumes, the average geometric scale variable of the cells can be introduced to represent the changing process of the myocardial cells under the influence of an external electromagnetic field. A variable particularly relevant to the present invention is the average longitudinal length of the myocardial cells, i.e., r(t), which is proven to be directly proportional to the myocardial capacitance measured under the external field. Based on this, the average longitudinal length of the myocardial cells and its change can be calculated by measuring the capacitance. From the change of the average longitudinal length of the myocardial cells, a method describing the overall longitudinal elasticity of the heart can be given. The overall longitudinal elasticity of the heart can be described by the relative change rate of the myocardial capacitance over time under an external electric field.

The most simplified model is to replace the myocardial cells with an equivalent sphere in the direction of the external electromagnetic field. At this time, the average longitudinal length r(t) can be regarded as the average contraction radius of the myocardial cells. Under the external electromagnetic field, the capacitance of a cell can be estimated with the following formula:

$$C(t) = 4\pi\varepsilon_0 \times r(t)$$

r(t) is the equivalent average contraction radius of the myocardial cells, that is, the average longitudinal length, which is a time variable. $\varepsilon_0$ is the cell's magnetic permeability. In general, the capacitance is also directionally proportional to the average longitudinal length of the myocardial cells, and the proportional coefficient is related to the geometrical shape and the magnetic permeability of the myocardial cells. For the sake of simplicity, the equivalent sphere is used for illustration below.

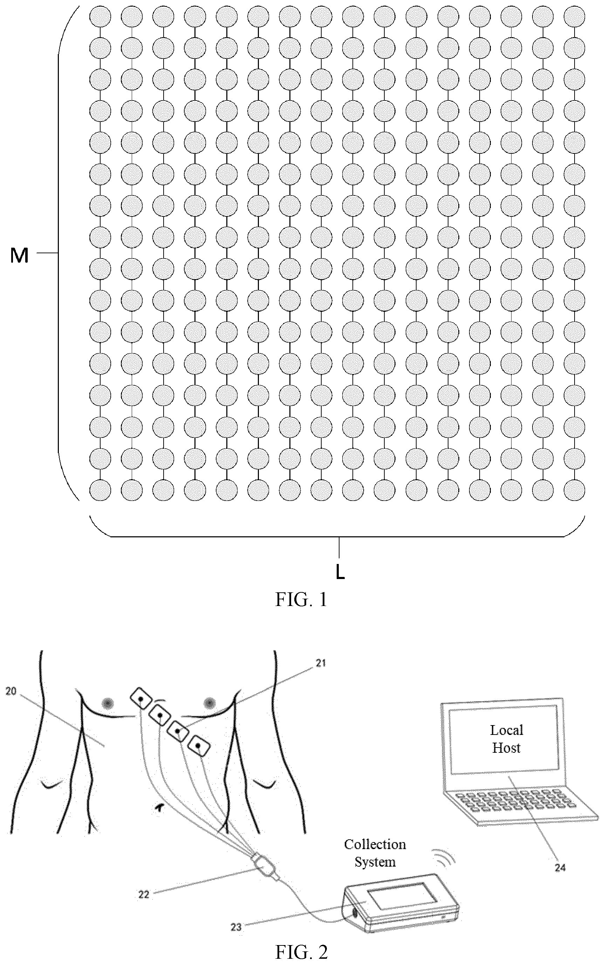

FIG. **1** is a schematic diagram of a two-dimensional abstract model of simulated myocardial cells provided by an embodiment of the present invention, which is supported by a plurality of myocardial cells' microstructures. Specifically, when the heart cells are in their normal positions, since the heart's structural cells restrict the muscle cells in space, it can be considered that the myocardial cells are connected in series and parallel modes. In an alternative embodiment, it is assumed that M cells are connected in series to form chains in the longitudinal direction, and L chains are in parallel connection in total. At the same time, the myocardial cells are replaced with an equivalent sphere in the direction of the external electromagnetic field. By the time, the average longitudinal length r(t) can be regarded as the average contraction radius of the myocardial cells, and the capacitance of a cell under the external electromagnetic field can be estimated by the following formula:

$$C(t) = 4\pi\varepsilon_0 r(t)\frac{L}{M}$$

Wherein, r(t) is the equivalent average contraction radius of the myocardial cells, that is, the average longitudinal length, which is a time variable, and $\varepsilon_0$ is the cell's magnetic permeability. It can be seen that under normal circumstances, C(t) and r(t) have a linear relationship, that is, the capacitance is directly proportional to the average longitudinal length of the myocardial cells, and the proportional coefficient is related to the geometric shape and the magnetic permeability of the myocardial cells. In an abnormal state, the position and size of r(t) will change, or the abnormal cells exhibit different magnetic permeabilities, resulting in the change of C(t), which also have different changing patterns.

FIG. 2 is an overall frame diagram of a part of the system provided by another embodiment of the present invention. Specifically, a human or animal body 20 is connected to a collection system 23 through electrodes or contacts 21 and cables 22. In an alternative embodiment, the voltage signals modulated by the human or animal body 20 are transmitted to the collection system 23 through the electrodes or contacts 21, and the collection system 23 processes the voltage signals and transmits them to a host 24 for further analysis. In an alternative embodiment, the host 24 comprises a human-computer interaction interface for receiving or transmitting external commands.

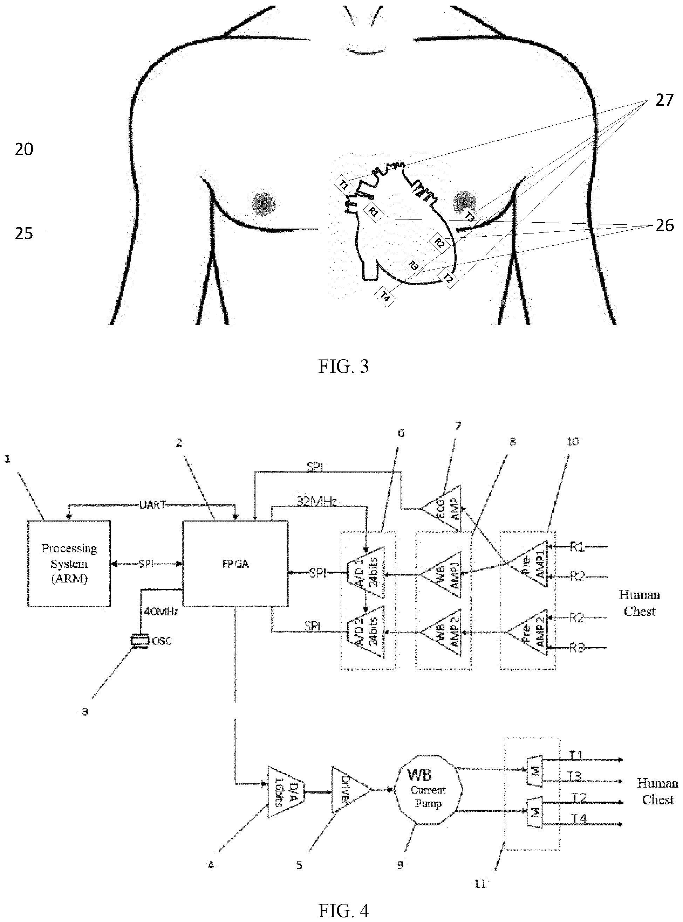

FIG. 3 is a schematic diagram illustrating the placement of the transmitting and receiving electrodes provided by another embodiment of the present invention. Specifically, 25 represents the heart tissue in the thoracic cavity of a human or animal body, and the transmitting electrodes 27 and the receiving electrodes 26 are all located at the skin directly above the heart tissue 25. In an alternative embodiment, the transmitting electrodes 27 comprise two pairs of electrodes T1-T2, and T3-T4. Each pair of the transmitting electrodes is driven in a time-sharing manner and are independent of each other. Electrodes T1 and T2 are respectively aligned to the outer edges of both longitudinal ends of the heart tissue 25, and electrodes T3 and T4 are respectively aligned to the outer edges of the both transverse ends of the heart tissue 25. The wideband current signals enter the human or animal body 20 from the transmitting electrodes 27. The receiving electrodes 26 comprise 3 electrodes R1, R2 and R3. All of them are aligned to the heart tissue 25 and located among the transmitting electrodes 27, for detecting the wideband voltage signals. In an alternative embodiment, electrodes R1 and R2 or electrodes R1 and R3 respectively constitute a longitudinal receiving pair, and electrodes R2 and R3 constitute a transverse receiving pair. The system can comprise these two receiving circuit pairs, so as to detect the heart tissue's motion changes in two directions.

FIG. 4 is a schematic diagram of a circuit structure of the system provided by another embodiment of the present invention. Specifically, the system can not only receive voltage signals, but also transmit current signals to the human or animal body and its tissues. In an alternative embodiment, wideband signals are generated from the frequency domain to the time domain in the integrated circuit (IC) of a microprocessor 1 or a field programmable gate array (FPGA) 2. If the wideband signals are updated infrequently, their time-domain signals can be stored in the system, and the FPGA 2 can continuously output the signals to a digital-to-analog converter (DAC) 4. In an alternative embodiment, in order to reduce analog distortion, the DAC typically operates at a high speed, for example, more than 16 times of the Nyquist rate. The output signals of the DAC 4 are amplified to drive the wideband current pump 9.

In an alternative embodiment, the output of the wideband current pump 9 is connected to the input of the analog switch 11, and the outputs of the analog switch 11 are connected to the transmitting electrode pair T1-T2, or T3-T4 respectively. Thus, the current signals are transmitted to the human or animal body.

In an alternative embodiment, the receiving electrode pair R1-R2, or pair R1-R3 may simultaneously or non-simultaneously receive signals from the long-axis direction of the heart. Meanwhile, the receiving electrode pair R2-R3 may receive signals from the short-axis of the heart.

In an alternative embodiment, the voltage signals modulated by the human or animal body are amplified by a preamplifier array 10. All outputs from the preamplifier array 10 are inputted to a wideband amplifier array 8, among which one output is also connected to a dedicated ECG amplification collector 7, to obtain ECG signals. The ECG signals are transmitted to the FPGA 2. The wideband amplifier array 8 outputs the signals to an analog-to-digital converter (ADC) 6. This embodiment uses a high-speed and high-resolution ADC. Then the ADC 6 converts the analog signals into digital signals and sends them to the FPGA 2.

In an alternative embodiment, changes in the cardiovascular system of the human body may cause an impedance change of 0.2 %, that is, the dynamic range is about -54dB. If the result of the received signal requires 1 % resolution, then the required dynamic range is 94 dB, which is about 16 bits. Therefore, the minimum requirement of the DAC used in this embodiment is 16 bits.

In an alternative embodiment, as the analog filter will change the phase response, a digital correction must be performed to calculate the human body phase response. This embodiment does not use an analog filter, but an oversampling DAC. The oversampling DAC's high rate will greatly reduce the dependence on the analog filter. The oversampling rate can be 16 times the Nyquist rate or higher.

In an alternative embodiment, the signal acquisition adopts a delta-sigma ADC, considering that the signal acquisition has higher requirements than signal generation, but oversampling like a DAC requires high hardware performance and resources, and the effect is not obvious if they are modulated signals. The delta-sigma ADC requires superposition and its sampling rate is not high. Specifically, when its sampling rate increases, its bit resolution will decrease. In an alternative embodiment, due to human differences, the dynamic changing range of the ADC needs to be considered. About 3 bits are reserved for this change, while at least one bit is reserved to prevent saturation. In its specific implementation, the ADC will have a minimum as 20 bits, in order to maintain the same dynamic range as the DAC, therefore a full-speed 24-bit sigma-delta ADC has a dynamic range of about 20 bits.

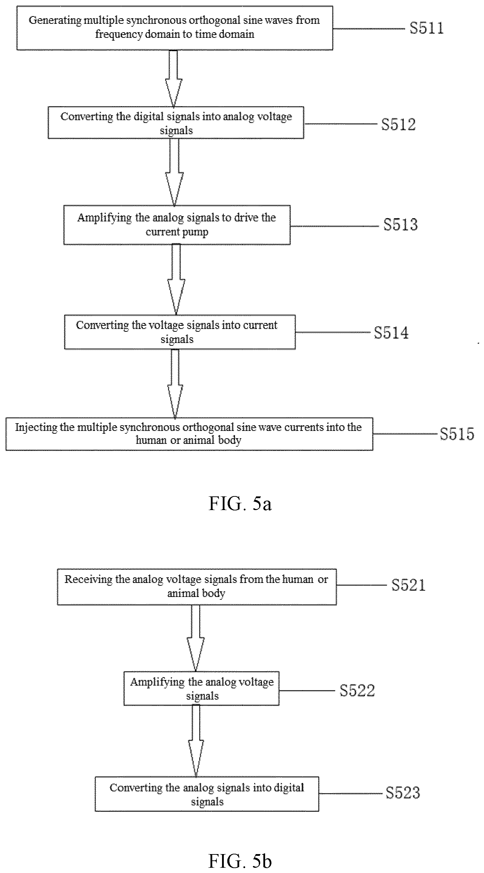

FIGS. 5a-5d are flowcharts of a method provided by another embodiment of the present invention, which specifically comprises signal generation, signal acquisition, and signal processing. In an alternative embodiment, as shown in FIG. 5a, the signal generation comprises generating multi-frequency synchronous orthogonal sine wave digital signals from the frequency domain to the time domain S511, converting the digital signals into analog signals S512, amplifying the analog signals to drive the current pump S513, converting the voltage signals into current signals S514, and injecting the multi-frequency synchronous orthogonal sine wave current into the human or animal body to be tested S515.

In an alternative embodiment, as shown in FIG. 5b, the signal acquisition specifically comprises receiving analog voltage signals from a human or animal body S521 and amplifying them S522, and converting the analog signals into digital signals S523.

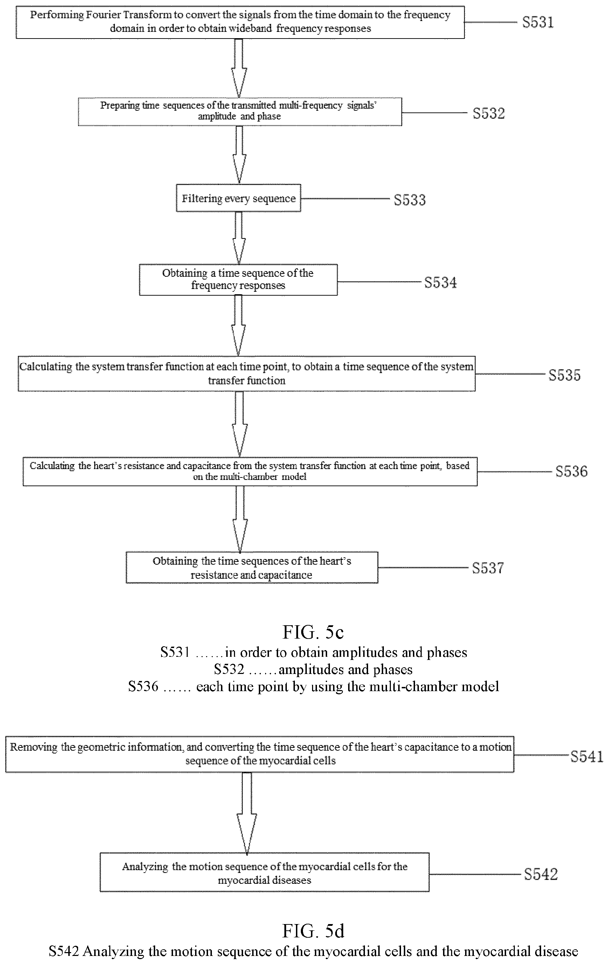

In an alternative embodiment, as shown in FIG. 5c, after the signals are collected, a Fourier Transform is performed to convert the signals from the time domain to the frequency domain in order to obtain wideband frequency responses S531, and these wideband frequency responses are time-varying. Frequency correction and filtering are later performed on these frequency responses, to eliminate distortion and noises S532-S534. These corrected and filtered frequency responses are used to calculate the system transfer function S535, which is also a time-varying sequence. According to the coefficient decomposition of the system transfer function, we can obtain the heart's resistance and capacitance S536. Then the resistance and capacitance sequences are filtered for the next processing S537. That is, the capacitance of the heart is directly related to the size of the myocardial cells.

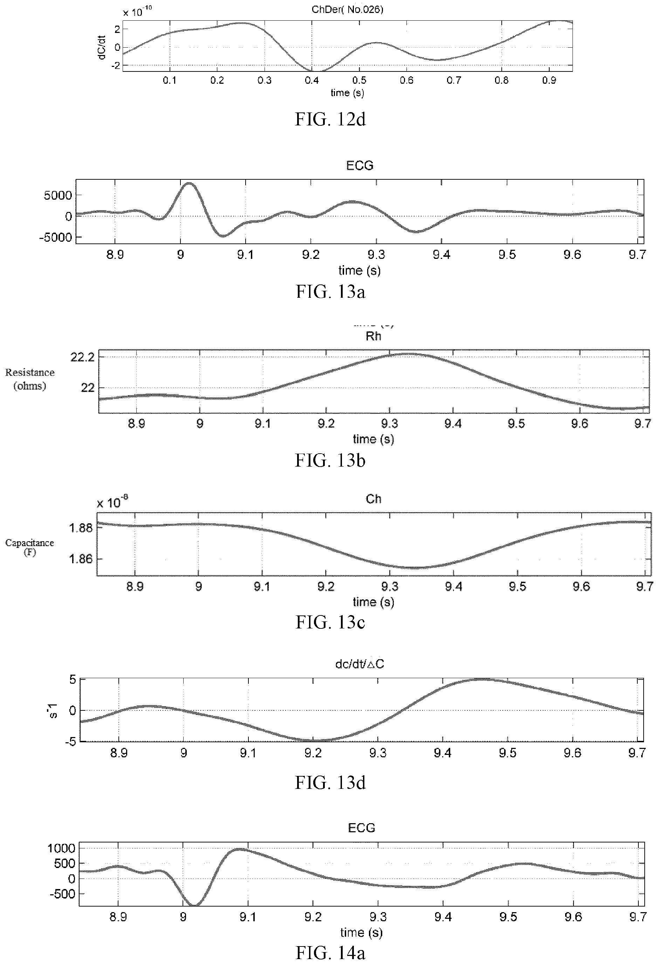

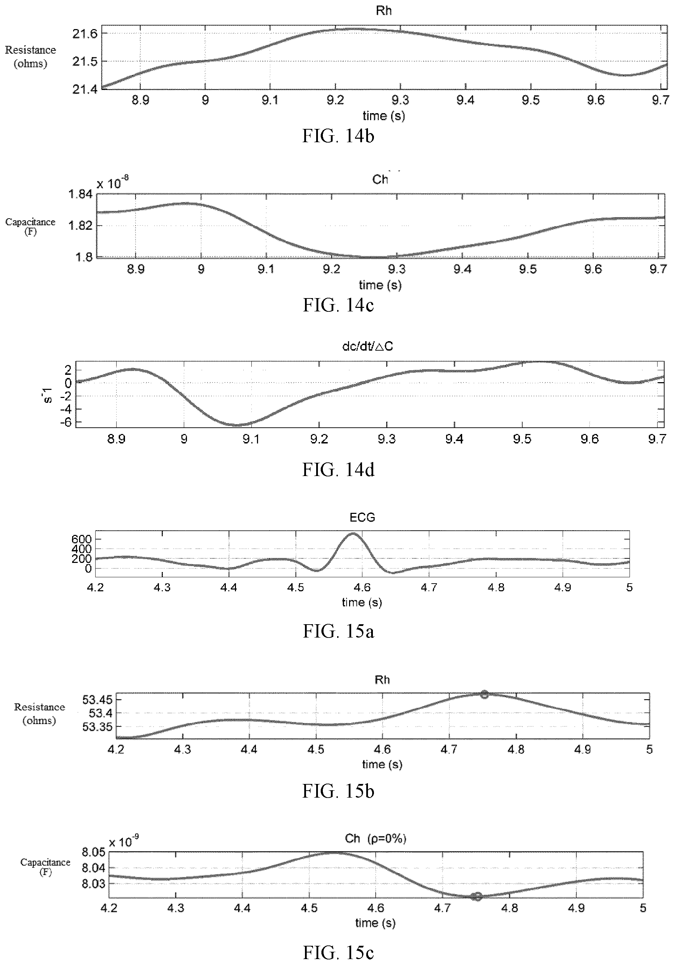

In an alternative embodiment, as shown in FIG. 5d, there is also geometric information in the capacitor, which should be removed S541. In the specific implementation of this embodiment, the time derivative of the capacitance is divided by the capacitance fluctuation in one cardiac cycle, that is, dc/dt/$\Delta$c. This method is related to the specific parameters. For example, in FIG. 13d and FIG. 14d, after the geometric information is removed, only information about the changes in the radius of the myocardial cells is left. It represents the change in myocardial cells during the cardiac cycle. On such as basis, further analysis and machine learning can be performed S542. The resistance of the heart is more complex, which includes the resistance of the blood in chambers and in the myocardial tissue. However, since the blood change in the heart is dominant, the resistance can be directly used to calculate the blood flow.

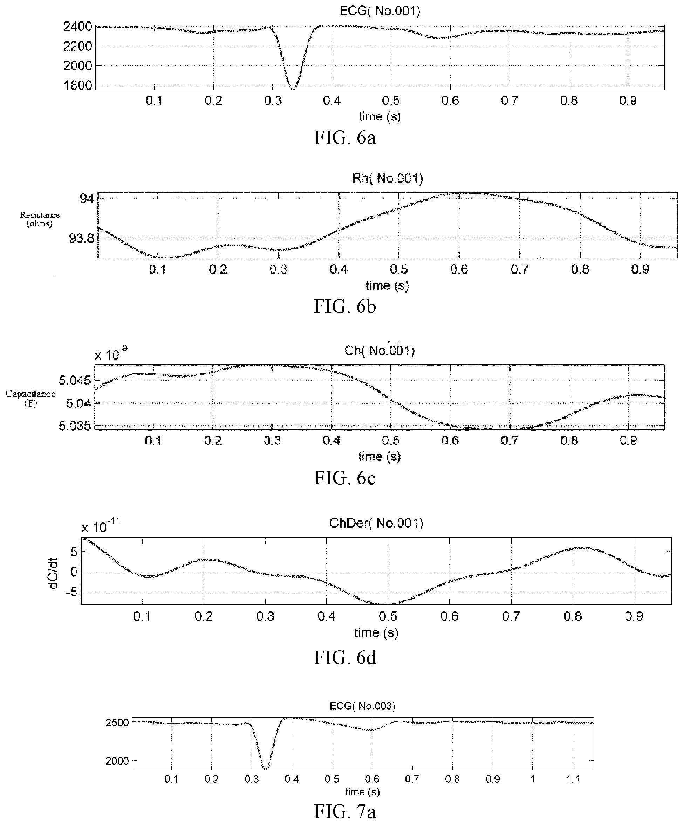

FIGS. 6a-6d are a young man's electrocardiogram, curves of his heart's resistance and capacitance over time, and the derivative of the capacitance curve provided by another embodiment of the present invention. This is the data of a normal person. Specifically, FIG. 6a is the electrocardiogram (ECG), FIG. 6b is the heart resistance curve, FIG. 6c is the myocardial capacitance curve, and FIG. 6d is the derivative change curve of the myocardial capacitance.

In the alternative embodiment, the electrocardiogram is not a standard pattern, but is obtained by simultaneous detection on the electrodes that measure the heart voltage signals. The heart resistance comes from the blood in the chambers and the myocardial tissue. At the end of diastole, the chamber has the highest blood volume and thus the minimum resistance. At the end of the systole, the situation is reversed. This is completely consistent with the actual data, so the blood resistance is dominant in the displayed heart resistance. At the end of diastole, myocardial cells relax and have the largest cell volume. Thus, the capacitance reaches its peak value. At the end of systole, the volume of the myocardial cells is the smallest and so is the capacitance. The capacitance curve in this cardiac cycle does not fully recover to the most diastolic level. There may be two reasons for this. The first is interference; the second is that the diastolic process has its randomness; and it is not that every cycle is the same and can be restored to the maximum position, which means that the peaks may vary. From the heart resistance curve, his heart volume starts to decrease (systole) from the R wave until the T wave occurs, and then it begins to increase (diastole). This is completely consistent with the myocardium's bioelectric activity in polarization and depolarization. From his heart's capacitance curve, the myocardial cells begin to shrink (systole) from the R wave, and until the T wave ocurrs, they begin to grow (diastole). It does not return to the maximum point of diastole, which is due to the randomness of diastole. From the volume of the heart and the volume change of the myocardial cells, the heart pumping and the myocardial work can be estimated, that is, the characteristics of the mechanical activity of biological tissues can be estimated based on their electrical activities.

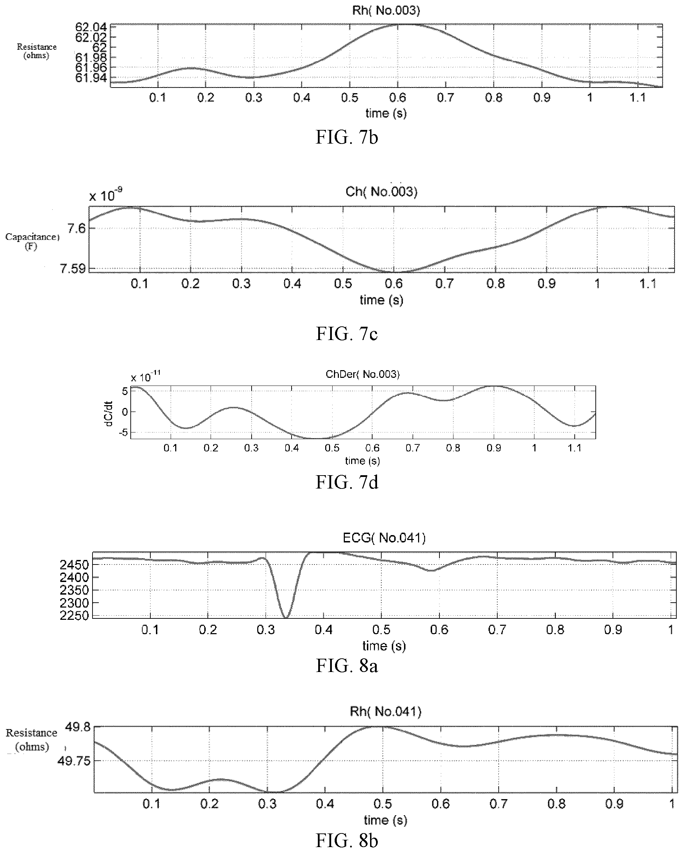

FIGS. 7a-7d are data of a normal middle-aged male provided by another embodiment of the present invention. FIG. 7a is the electrocardiogram (ECG), FIG. 7b is the heart resistance curve, FIG. 7c is the myocardial capacitance curve, and FIG. 7d is the derivative of the myocardial capacitance curve. By comparing FIGS. 7a-7d with FIGS. 6a-6d, it can be found that the starting point of increase (diastole) of the myocardial cell volume in FIGS. 7a-7d is at the peak of the T wave, which is earlier than that in FIGS. 6a-6d. It is speculated that as the age increases, the elasticity of the myocardium decreases, the contraction period becomes shorter, and the starting point of myocardial diastole is earlier and earlier. The subject's myocardial relaxation is fully recovered during this cycle.

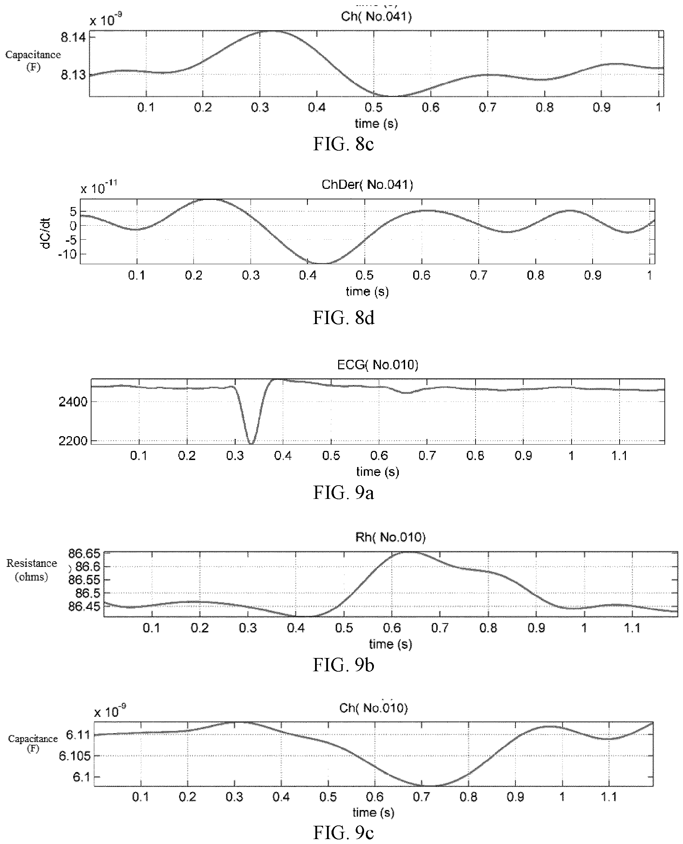

FIGS. 8a-8d are data of an elderly woman provided by another embodiment of the present invention, wherein FIG. 8a is the electrocardiogram (ECG), FIG. 8b is the heart resistance curve, FIG. 8c is the myocardial capacitance curve, and FIG. 8d is the derivative of the myocardial capacitance curve. The subject's blood pressure is relatively high, and premature beats are identified. From the heart resistance curve, the subject's systole is normal, but it is completed long before the myocardial repolarization. After the repolarization, the heart volume does not change much in this cycle, that is, there is not much blood filling. From the capacitance curve, the myocardium completes the contraction well before the T wave, and begins to relax, but the relax is very slowly, and does not return to the maximum relax point. The systole is too fast and the diastole is slow, thus it is speculated that the myocardial tissue is aging.

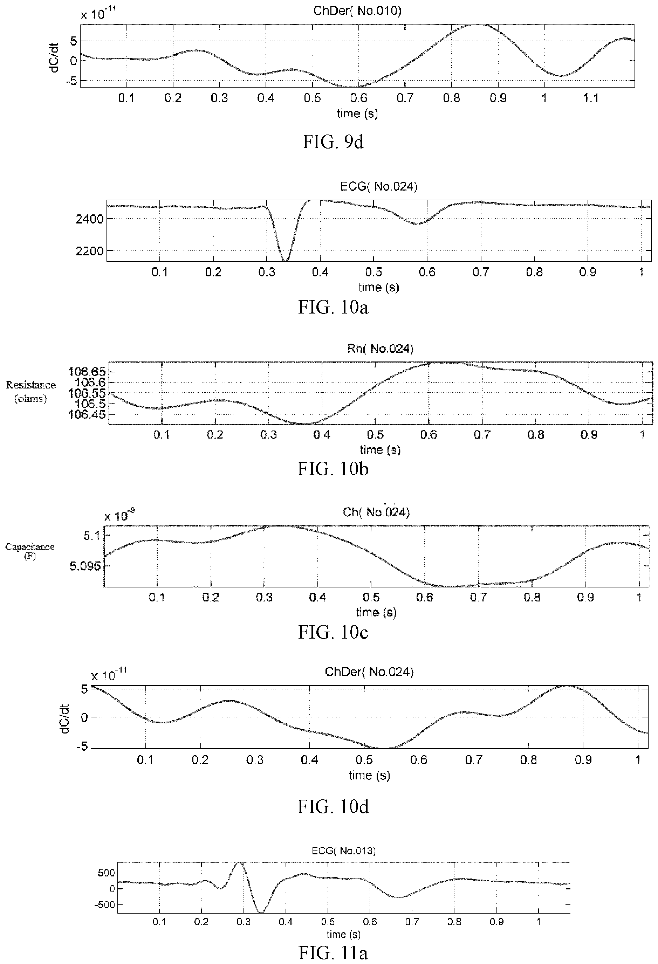

FIGS. 9a-9d are data of an elderly woman provided by another embodiment of the present invention, wherein FIG. 9a is the electrocardiogram (ECG), FIG. 9b is the heart resistance curve, FIG. 9c is the myocardial capacitance curve, and FIG. 9d is the derivative of the myocardial capacitance curve. From the resistance curve, the heart volume contraction of the subject lags behind the R wave a lot, that is, the left ventricular pressure is not enough, so the aorta cannot be opened, and no blood is ejected. Then the aorta opens, the blood in the heart decreases, and the contraction is completed just before the peak of the T wave. Then the heart is filled up normally. From the capacitance curve, the starting point of myocardial contraction is normal, but the myocardium seems to be weak, the volume of myocardial cells changes very little, and it increases later. The myocardial relaxation ends at the T wave, and returns to the maximum relax point. It can be seen from this that the minimum point of the heart volume and the minimum point of the myocardial volume are not necessarily at the same time point.

FIGS. 10a-10d are data of an elderly woman provided by another embodiment of the present invention, wherein FIG. 10a is the electrocardiogram (ECG), FIG. 10b is the heart resistance curve, FIG. 10c is the myocardial capacitance curve, and FIG. 10d is the derivative of the myocardial capacitance curve. From the resistance curve, the heart volume contraction of the subject lags behind the R wave, and is completed just after the peak of the T wave. Then the heart starts filling, but the filling is seriously lagging behind. From the capacitance curve, the starting point of myocardial contraction is normal, the process of myocardial contraction is basically normal, and it reaches the minimum just after the T wave; but the myocardial relaxation is seriously delayed, and it can basically recover in the end.

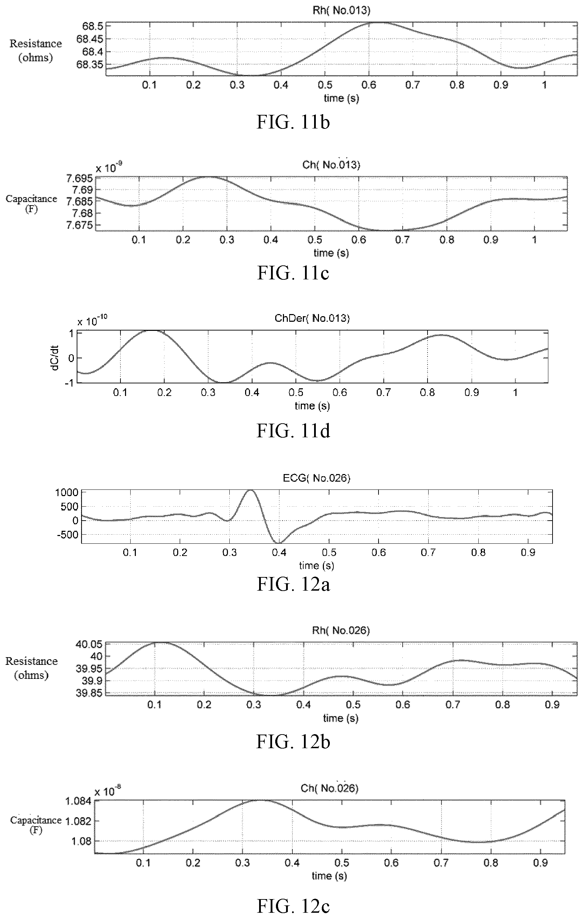

FIGS. 11a-11d are data of an elderly woman provided by another embodiment of the present invention. FIG. 11a is the electrocardiogram (ECG), FIG. 11b is the heart resistance curve, FIG. 11c is the myocardial capacitance curve, and FIG. 11d is the derivative of the myocardial capacitance curve. From the resistance curve, the systole lags slightly and is completed before the peak of the T wave. Then the heart relaxes. From the capacitance curve, the starting point of the myocardial contraction is normal, but the myocardial contraction is divided into two regions, which is more obvious on the derivative curve of the capacitance. Therefore, the states of the myocardial cells are not uniform. The myocardial cells can also relax and recover. It can be determined that the subject's myocardium is defective.

FIGS. 12a-12d are data of an elderly woman provided by another embodiment of the present invention, wherein FIG. 12a is the electrocardiogram (ECG), FIG. 12b is the heart resistance curve, FIG. 12c is the myocardial capacitance curve, and FIG. 12d is the derivative of the myocardial capacitance curve. From the resistance curve, the starting point of systole is normal, and the T wave is not obvious. The subject's systole is divided into two parts, and this cardiac cycle does not reach the maximum contraction. The starting point of myocardial cell contraction is normal, however, the myocardial cell contraction is divided into two stages, and the contractions are not consistent, indicating that the myocardial cells cannot coordinate to do work. The diastole is severely delayed, but it can be restored to its maximum state. It can be judged that the subject suffers from a heart disease.

FIGS. 13a-13d and FIGS. 14a-14d are data of two persons provided by another embodiment of the present invention, wherein, FIG. 13a and FIG. 14a are the electrocardiograms (ECG), FIG. 13b and FIG. 14b are the heart resistance curves, FIG. 13c and FIG. 14c are the myocardial capacitance curves, and FIG. 13d and FIG. 14d are time curves of the relative change rate of myocardial capacitance. FIG. 13d and FIG. 14d show the time curves of ECG, capacitance and resistance, and the equivalent deformation rate ($S^{-1}$) of the myocardial cells, or the relative change rate $\dot{\varepsilon}$ of the capacitance is defined as:

$$\dot{\varepsilon} = \frac{dc(t)/dt}{c_{pp}}$$

$dc(t)/dt$ is the time derivative of the capacitance, and $c_{pp}$ is the capacitance's peak-to-peak value in this cardiac cycle.

Or, the relative change $\varepsilon$ of the capacitance is defined as:

$$\varepsilon = \frac{\Delta c(t)}{c_{pp}} \times 100\%$$

$\Delta c(t)$ is the difference of capacitances at two points in time.

It can be seen from the figures that, for a normal person, the heart volume change is completely consistent with change of the myocardial cell volume. The myocardial cells' equivalent deformation rate ($S^{-1}$), or the relative change rate of the capacitance in this embodiment, and the relative change of the capacitance, are two measurement parameters.

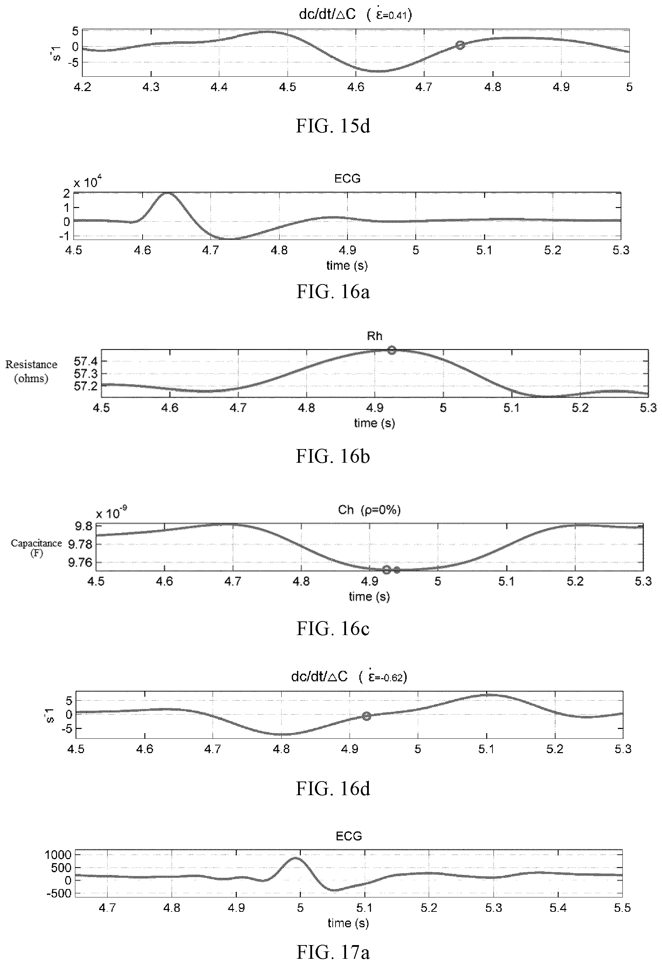

FIGS. 15a-15d and FIGS. 16a-16d are data of two normal persons provided by another embodiment of the present invention, wherein FIG. **15**a and FIG. **16**a are the electrocardiograms (ECG), FIG. 15b and FIG. 16b are heart resistance curves, FIG. 15c and FIG. 16c are the myocardial capacitance curves, FIG. 15d and FIG. 16d are time curves of the relative change rate of myocardial capacitance. As shown in the figures, the circle marks are the moments when the heart volume is the smallest, and the solid dot are the moments when the myocardial cell volume is the smallest. In the motion of the myocardial tissue of a normal person, the circle and the solid point basically overlap. ρ in the myocardial capacitance curve, i.e., the relative change of the myocardial capacitance, is defined as substracting the capacitance value at the moment when the heart volume is the minimum from the minimum capacitance value, and then dividing the result by the peak-to- peak value of the capacitance in this cardiac cycle, which is as follows:

$$\rho = \frac{c(t_{circle}) - c(t_{dot})}{c_{pp}} \times 100\%$$

Wherein, $c(t_{circle})$ is the capacitance at the moment when the heart volume is the smallest, $c(t_{dot})$ is the capacitance at the moment when the myocardial volume is the smallest, and $c_{pp}$ is the capacitance's peak-to-peak value in this cardiac cycle. The relative change of the myocardial capacitance corresponds to the change of tensor in ultrasound. In ultrasound, when the aortic valve is closed, the equivalent deformation (%) and the equivalent deformation rate ($S^{-1}$) of the tissue are also detected. Although there is no direct information about the closure of the aortic valve in this embodiment, the moment of the minimum value of the heart volume (i.e., the maximum value of the resistance) can be regarded as the moment when the aortic valve is closed. The relative change rate of the capacitance ($S^{-1}$) measured at this moment should be consistent with the equivalent deformation rate ($S^{-1}$) in ultrasonic testing, both approaching zero. The capacitance's relative change (%) measured at this point in time should be consistent with the tensor deformation in ultrasonic testing, both approaching zero. In ultrasound, the maximum of the equivalent deformation rate ($S^{-1}$) for a normal person in the systole is 1 ($S^{-1}$). In other words, at the moment of the minimum heart volume or the maximum resistance, the relative change (ρ) of capacitance and the equivalent deformation rate ($S^{-1}$) are both approaching zero. The relative change (ρ) of capacitance corresponds to the tensor deformation at the moment of the closure of the aorta in ultrasonic Doppler tissue imaging.

In an alternative embodiment, with the help of high sampling rate and high precision, this embodiment can obtain more information, such as using a waveform analysis method, combining P, R, and T waves in the electrocardiogram, and further combined with statistical models, and the curve characteristics of the resistance and the capacitance, one can totally analyze the elasticity of the tissue from the perspective of deformation mechanics, that is, analyze the contraction and extension of the myocardial cells from the change process of their average longitudinal length, such as calculating the elasticity of the myocardium and its ability to do work from the speeds of contraction and relaxation.

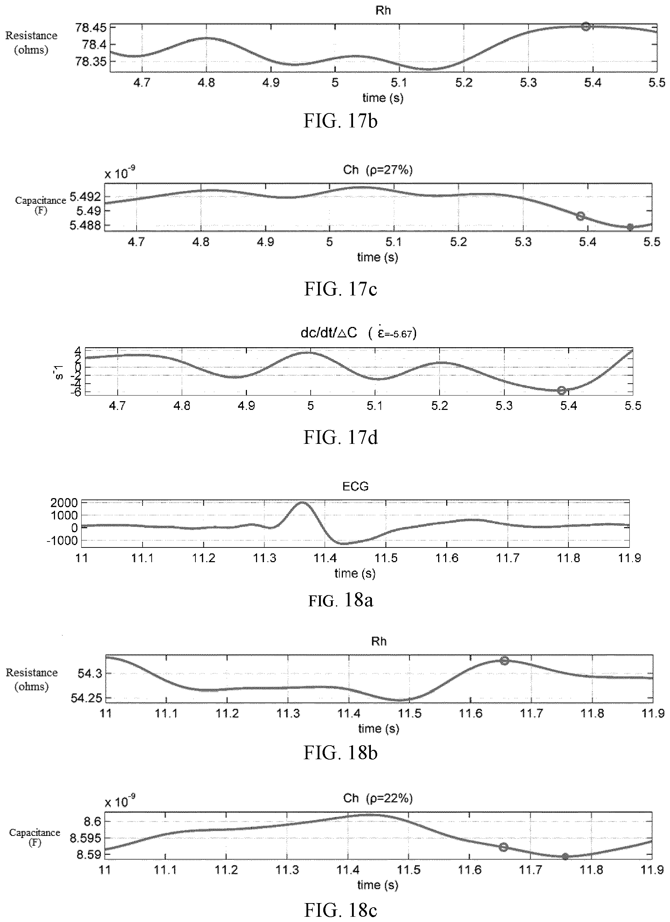

FIGS. 17a-17d are data of a person with an abnormal heart tissue provided by another embodiment of the present invention, wherein, FIG. **17**a is the electrocardiogram (ECG), FIG. 17b is the heart resistance curve, FIG. 17c is the myocardial capacitance curve, and FIG. **17**d is the time curve of the relative change rate of myocardial capacitance. The circle marks in the figures are the moments when the heart volume is the smallest, and the solid dots are the moments when the myocardial cell volume is the smallest. According to calculations, the results of ρ (27%) and $\dot{\varepsilon}$ (-5.67) both show that the heart tissue is abnormal.

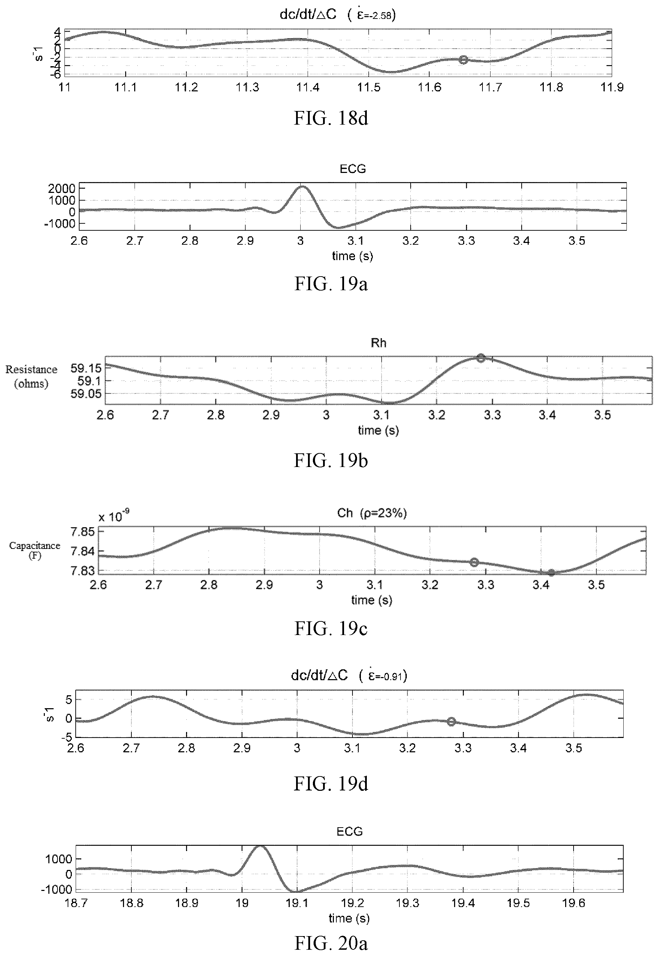

FIGS. 18a-18d are data of a person with an abnormal heart tissue provided by another embodiment of the present invention, wherein, FIG. **18**a is the electrocardiogram (ECG), FIG. 18b is the heart resistance curve, FIG. 18c is the myocardial capacitance curve, and FIG. **18**d is the time curve of the relative change rate of myocardial capacitance. The circle marks in the figures are the moments when the heart volume is the smallest, and the solid dots are the moments when the myocardial cell volume is the smallest. According to calculations, the result of ρ (22%) shows that the heart tissue is abnormal, and the result of $\dot{\varepsilon}$ (-2.6) shows that the heart tissue is slightly abnormal.

FIGS. 19a-19d are data of a person with an abnormal heart tissue provided by another embodiment of the present invention, wherein, FIG. **19**a is the electrocardiogram (ECG), FIG. 19b is the heart resistance curve, FIG. 19c is the myocardial capacitance curve, and FIG. **19**d is the curve of the relative change rate of myocardial capacitance over time. The circle marks in the figures are the moments when the heart volume is the smallest, and the solid dots are the moments when the myocardial cell volume is the smallest. According to calculations, the result of ρ (23%) shows that the heart tissue is abnormal, and the result of $\dot{\varepsilon}$ (-0.9) shows that the heart tissue is basically normal.

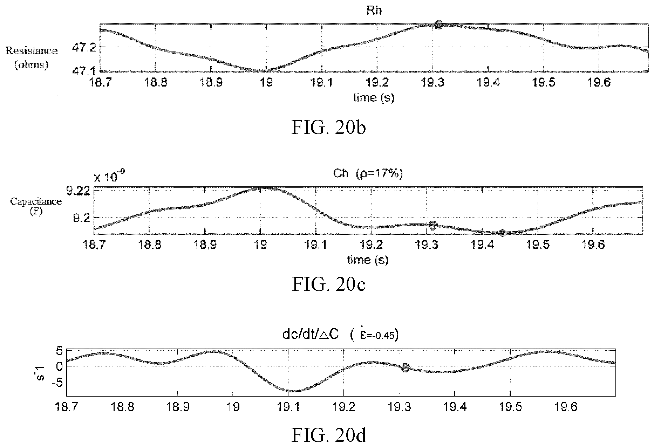

FIGS. 20a-20d are data of a person with an abnormal heart tissue provided by another embodiment of the present invention, wherein, FIG. **20**a is the electrocardiogram (ECG), FIG. 20b is the heart resistance curve, FIG. 20c is the myocardial capacitance curve, and FIG. **20**d is the time curve of the relative change rate of myocardial capacitance. The circle marks in the figures are the moments when the heart volume is the smallest, and the solid dots are the moments when the myocardial cell volume is the smallest. According to calculations, the result of ρ (17 %) shows that the heart tissue is abnormal, and the result of $\dot{\varepsilon}$ (-0.45) shows that the heart tissue is basically normal.

The above descriptions describe the specific embodiments. The scope of the present invention is not limited to the content in the specification, but is determined according to the scope of the claims.

**Claims**

1. A non-invasive method for measuring motion characteristics of a myocardial tissue, wherein the method comprises:

   transmitting a plurality of generated synchronous orthogonal, phase controllable and adjustable alternating currents with different frequencies into an organism so as to generate a plurality of synchronous periodic AC voltage signals with different frequencies;
   receiving the periodic AC voltage signals modulated by changes in the organism's heart tissue to obtain organism's frequency responses; calculating resistances and capacitances of the heart tissue according to the frequency responses;

   and estimating the motion characteristics of the myocardial tissue according to the resistances and the capacitances, wherein the estimating the motion characteristics of the myocardial tissue according to the resistances and the capacitances comprises:

   calculating an average longitudinal length of myocardial cells and its change according to the capacitances, and/or calculating heart pumping blood flow according to the resistances;
   and obtaining an overall longitudinal elastic state of the heart according to the average longitudinal length of the myocardial cells and its change and/or the heart pumping blood flow.

2. The method according to claim **1,** wherein the calculating resistances and capacitances of the heart tissue according to the frequency responses comprises, obtaining a system transfer function of the organism according to the frequency responses, and performing multi-chamber modeling to separate the heart tissue and peripheral tissues.

3. The method according to claim **1,** wherein the method further comprises, analyzing a systole speed, time, intensity and pattern of the heart tissue, and/or a diastole speed, time, recovery and pattern of the heart tissue according to a slope value of changes of the overall longitudinal elastic state of the heart, their delay to an R wave, a peak-to-peak value, and a change curve and its derivative's shape of the average longitudinal length of the myocardial cells.

4. The method according to claim **1,** wherein the obtaining the organism's frequency responses comprises, calculating a frequency response estimation value of a specific frequency every 0.25 to 5 milliseconds.

5. The method according to claim 1, wherein the calculating the average longitudinal length of myocardial cells and its change according to the capacitances comprises:

   detecting the average longitudinal lengths of the myocardial cells and its change over time at a rate of 200 to 4000 times per second;
   and processing the time sequence of the change over time of the average longitudinal length of the myocardial cells using a digital signal processing method, wherein the digital signal processing method comprises digital filtering, Fast Fourier Transform (FFT), and time domain and frequency domain analysis.

6. The method according to claim **5,** wherein the method further comprises, referring to an electrocardiogram having the same time sequence to analyze the change sequence of the average longitudinal length of the myocardial cells, wherein the referring comprises comparing the electrocardiogram with the change sequence of the average longitudinal lengths of the myocardial cells for their cardiac cycles, systolic and diastolic phases, and/or the boundaries thereof.

7. The method according to claim **2,** wherein the performing multi-chamber modeling to separate the heart tissue and peripheral tissues comprises, modeling each chamber as parallel resistor and capacitor, and multiple chambers being connected in series or in parallel.

8. A system for implementing a method according to any one of claims 1 to 7, wherein the system comprises a terminal and at least one processor, wherein the terminal comprises:

   a generator configured to generate a plurality of synchronous orthogonal, phase controllable and adjustable, and periodic alternating currents with different frequencies;
   and one or more sensors configured to transmit the periodic alternating currents into an organism to generate a plurality of periodic AC voltage signals with different frequencies, and receive the periodic AC voltage signals modulated by changes in the heart tissue of the organism to obtain the organism's frequency responses; wherein the processor is configured to calculate resistances and capacitances of the heart tissue according to the frequency responses, and to estimate the motion characteristics of the myocardial tissue according to the resistances and the capacitances, wherein the processor is configured to calculate an average longitudinal length of myocardial cells and its change according to the capacitances, and/or calculating heart pumping blood flow according to the resistances, and obtain an overall longitudinal elastic state of the heart according to the average longitudinal length of the myocardial cells and its change and/or the heart pumping blood flow.

9. The system according to claim **8,** wherein the sensor is configured to collect single or multiple pieces of data from different parts.

10. The system according to claim **8,** wherein the system further comprises a database for storing processing results and data of the processor or processors, and the processor or processors can retrieve the database.

11. The system according to claim **8,** wherein the processor or processors can be remote, and can be used for remote observation of the system's work in a real-time mode.

12. The system according to any one of claims **8-11,** wherein the terminal further comprises a man-machine interface for controlling the system and/or displaying results.

**Patentansprüche**

1. Nicht-invasives Verfahren zur Messung der Bewegungseigenschaften eines Myokardgewebes, wobei das Verfahren Folgendes umfasst:

   Übertragen einer Vielzahl von erzeugten synchronen orthogonalen, phasensteuerbaren und einstellbaren Wechselströmen mit unterschiedlichen Frequenzen in einen Organismus, um eine Vielzahl von synchronen periodischen Wechselspannungssignalen mit unterschiedlichen Frequenzen zu erzeugen;
   Empfangen von periodischen Wechselspannungssignalen, die durch Veränderungen im Herzgewebe des Organismus moduliert werden, um die Frequenzgänge des Organismus zu erhalten; Berechnung der Widerstände und Kapazitäten des Herzgewebes in Abhängigkeit von den Frequenzgängen;
und Schätzung der Bewegungseigenschaften des Myokardgewebes entsprechend den Widerständen und den Kapazitäten, wobei die Schätzung der Bewegungseigenschaften des Myokardgewebes in Abhängigkeit von den Widerständen und den Kapazitäten Folgendes umfasst:

   Berechnung der durchschnittlichen Längslänge der Myokardzellen und ihrer Änderung in Abhängigkeit von den Kapazitäten und/oder Berechnung des Blutflusses des Herzens in Abhängigkeit von den Widerständen;
   und Erreichen eines longitudinalelastischen Gesamtzustands des Herzens entsprechend der durchschnittlichen Längslänge der Myokardzellen und ihrer Veränderung und/oder des Herzpumpens des Blutflusses.

2. Verfahren nach Anspruch 1, wobei das Berechnen von Widerständen und Kapazitäten des Herzgewebes gemäß den Frequenzgängen umfasst, das Erlangen einer Systemübertragungsfunktion des Organismus gemäß den Frequenzgängen und das Durchführen einer Mehrkammermodellierung zur Trennung des Herzgewebes und des peripheren Gewebes.

3. Verfahren nach Anspruch 1, wobei das Verfahren ferner das Analysieren einer Systolegeschwindigkeit, Zeit, Intensität und Muster des Herzgewebes und/oder einer Diastolengeschwindigkeit, -zeit, -wiederherstellung und -muster des Herzgewebes entsprechend einem Steigungswert von Änderungen des gesamten longitudinalelastischen Zustands des Herzens umfasst, deren Verzögerung zu einer R-Welle, ein Spitze-zu-Spitze-Wert und eine Änderungskurve und die Form ihrer Ableitung der durchschnittlichen Längslänge der Myokardzellen.

4. Verfahren nach Anspruch 1, wobei das Ermitteln der Frequenzgänge des Organismus das Berechnen eines Frequenzgang-Schätzwertes einer spezifischen Frequenz alle 0,25 bis 5 Millisekunden umfasst.

5. Verfahren nach Anspruch 1, wobei das Berechnen der durchschnittlichen Längslänge von Myokardzellen und deren Änderung entsprechend den Kapazitäten Folgendes umfasst:

   Erkennung der durchschnittlichen Längslängen der Myokardzellen und ihrer Veränderung im Laufe der Zeit mit einer Geschwindigkeit von 200 bis 4000 Mal pro Sekunde;
   und Verarbeiten der zeitlichen Abfolge der Änderung der durchschnittlichen Längslänge der Myokardzellen über die Zeit unter Verwendung eines digitalen Signalverarbeitungsverfahrens, wobei das digitale Signalverarbeitungsverfahren digitale Filterung, schnelle FourierTransformation (FFT) und Zeitbereichs- und Frequenzbereichsanalyse umfasst.

6. Verfahren nach Anspruch 5, wobei das Verfahren ferner es umfasst, unter Bezugnahme auf ein Elektrokardiogramm mit der gleichen Zeitsequenz die Veränderungssequenz der durchschnittlichen Längslänge der Myokardzellen zu analysieren, wobei das Bezugsverfahren das Vergleichen des Elektrokardiogramms mit der Veränderungssequenz der durchschnittlichen Längslängen der Myokardzellen für ihre Herzzyklen umfasst, systolische und diastolische Phase und/oder deren Grenzen.

7. Verfahren nach Anspruch 2, wobei die Durchführung der Mehrkammermodellierung zur Trennung des Herzgewebes und des peripheren Gewebes Modellieren jeder Kammer als paralleler Widerstand und Kondensator modelliert umfasst, wobei mehrere Kammern in Reihe oder parallel geschaltet sind.

8. System zum Implementieren eines Verfahrens nach einem der Ansprüche 1 bis 7, wobei das System ein Endgerät und mindestens einen Prozessor umfasst, wobei das Endgerät Folgendes umfasst:

ein Generator, der konfiguriert ist, um eine Vielzahl von synchronen orthogonalen, phasensteuerbaren und einstellbaren und periodischen Wechselströmen mit unterschiedlichen Frequenzen zu erzeugen; und einen oder mehrere Sensoren, die so konfiguriert sind, dass sie die periodischen Wechselströme in einen Organismus übertragen, um eine Vielzahl von periodischen Wechselspannungssignalen mit unterschiedlichen Frequenzen zu erzeugen, und die periodischen Wechselspannungssignale zu empfangen, die durch Veränderungen im Herzgewebe des Organismus moduliert werden, um die Frequenzgänge des Organismus zu erhalten; wobei der Prozessor konfiguriert ist, um Widerstände und Kapazitäten des Herzgewebes entsprechend den Frequenzgängen zu berechnen und die Bewegungseigenschaften des Myokardgewebes entsprechend den Widerständen und Kapazitäten zu schätzen, wobei der Prozessor so konfiguriert ist, dass er eine durchschnittliche Längslänge von Myokardzellen und deren Änderung entsprechend den Kapazitäten berechnet, und/oder Berechnung des Herzpumpblutflusses gemäß den Widerständen und erhalten Sie einen longitudinalelastischen Gesamtzustand des Herzens entsprechend der durchschnittlichen Längslänge der Myokardzellen und ihrer Veränderung und/oder des Herzpumpblutflusses.

9. System nach Anspruch 8, wobei der Sensor so konfiguriert ist, dass er einzelne oder mehrere Datenstücke von verschiedenen Teilen sammelt.

10. System nach Anspruch 8, wobei das System ferner eine Datenbank zum Speichern von Verarbeitungsergebnissen und Daten des Prozessors oder der Prozessoren umfasst, und der oder die Prozessoren die Datenbank abrufen können.

11. System nach Anspruch 8, wobei der Prozessor oder die Prozessoren ferngesteuert sein können und zur Fernbeobachtung der Arbeit des Systems in einem Echtzeitmodus verwendet werden können.

12. System nach einem der Ansprüche 8 bis 11, wobei das Endgerät ferner eine Mensch-Maschine-Schnittstelle zur Steuerung des Systems und/oder zur Anzeige von Ergebnissen umfasst.

**Revendications**

1. Procédé non invasif pour mesurer des caractéristiques dynamiques d'un tissu myocardique, comprenant :

transmettre une pluralité de courants alternatifs générés qui sont orthogonaux synchrones, contrôlables et réglables en phase à différentes fréquences vers un organisme, afin de générer une pluralité de signaux de tension alternative périodiques synchrones à différentes fréquences ; recevoir les signaux de tension alternative périodiques modulés par des variations du tissu cardiaque de l'organisme pour obtenir des réponses en fréquence de l'organisme ; et calculer des résistances et des capacités du tissu cardiaque en fonction des réponses en fréquence ; et estimer des caractéristiques dynamiques du tissu myocardique en fonction des résistances et des capacités, l'estimation des caractéristiques dynamiques du tissu myocardique en fonction des résistances et des capacités comprenant :

calculer une longueur longitudinale moyenne des cellules myocardiques et sa variation en fonction des capacités, et/ou calculer le débit de pompage sanguin du coeur en fonction des résistances ; et obtenir un état élastique longitudinal global du coeur en fonction de la longueur longitudinale moyenne des cellules myocardiques et de sa variation et/ou du débit de pompage sanguin du coeur.

2. Procédé selon la revendication 1, dans lequel l'étape de calculer des résistances et des capacités du tissu cardiaque en fonction des réponses en fréquence comprend : obtenir une fonction de transfert systématique de l'organisme en fonction des réponses en fréquence, et effectuer une modélisation multichambre pour séparer le tissu cardiaque et les tissus périphériques.

3. Procédé selon la revendication 1, dans lequel le procédé comprend en outre : analyser une vitesse, un temps, une intensité et un profil de la systole du tissu cardiaque, et/ou une vitesse, un temps, une récupération et un profil de la diastole du tissu cardiaque en fonction d'une valeur de pente des variations de l'état élastique longitudinal global du coeur, de leur retard à une onde R, une valeur pic à pic, et une courbe de variation et la forme de sa dérivée de la longueur longitudinale moyenne des cellules myocardiques.

4. Procédé selon la revendication 1, dans lequel l'étape d'obtenir des réponses en fréquence de l'organisme comprend : calculer une valeur estimée de la réponse en fréquence d'une fréquence spécifique toutes les 0,25 à 5 millisecondes.

5. Procédé selon la revendication 1, dans lequel l'étape de calculer la longueur longitudinale moyenne des cellules myocardiques et sa variation en fonction des capacités comprend :

    détecter la longueur longitudinale moyenne des cellules myocardiques et sa variation avec le temps à un rythme de 200 à 4000 fois par seconde ; et
    traiter la séquence temporelle de la variation avec le temps de la longueur longitudinale moyenne des cellules myocardiques par une méthode de traitement de signaux numériques, la méthode de traitement de signaux numériques comprenant un filtrage numérique, une transformation de Fourier rapide (FFT) et une analyse de domaine temporel et de domaine fréquentiel.

6. Procédé selon la revendication 5, dans lequel le procédé comprend en outre, se référer à un électrocardiogramme ayant la même séquence temporelle pour analyser la séquence de variation de la longueur longitudinale moyenne des cellules myocardiques, l'étape de se référer comprenant : comparer l'électrocardiogramme avec la séquence de variation des longueurs longitudinales moyennes des cellules du myocarde pour leurs cycles cardiaques, phases systolique et diastolique, et/ou leurs limites.

7. Procédé selon la revendication 2, dans lequel la modélisation multichambre pour séparer le tissu cardiaque et les tissus périphériques comprend : modéliser chaque chambre en tant que résistance et condensateur parallèles, les plusieurs chambres étant connectées en série ou en parallèle.

8. Système de mise en oeuvre d'un procédé selon l'une quelconque des revendications 1 à 7, dans lequel le système comprend un terminal et au moins un processeur, dans lequel le terminal comprend :

    un générateur configuré pour générer une pluralité de courants alternatifs orthogonaux synchrones, contrôlables et réglables en phase et périodiques à différentes fréquences ; et
    un ou plusieurs capteurs configurés pour transmettre les courants alternatifs périodiques vers un organisme, afin de générer une pluralité de signaux de tension alternative périodiques à différentes fréquences, et recevoir les signaux de tension alternative périodiques modulés par des variations du tissu cardiaque de l'organisme pour obtenir des réponses en fréquence de l'organisme ;
    dans lequel le processeur est configuré pour calculer des résistances et des capacités du tissu cardiaque en fonction des réponses en fréquence, et pour estimer des caractéristiques dynamiques du tissu myocardique en fonction des résistances et des capacités, dans lequel le processeur est configuré pour calculer une longueur longitudinale moyenne des cellules myocardiques et sa variation en fonction des capacités, et/ou calculer le débit de pompage sanguin du coeur en fonction des résistances, et obtenir un état élastique longitudinal global du coeur en fonction de la longueur longitudinale moyenne des cellules myocardiques et de sa variation et/ou du débit de pompage sanguin du coeur.

9. Système selon la revendication 8, dans lequel le capteur est configuré pour collecter un ou plusieurs de données à partir de différentes parties.

10. Système selon la revendication 8, dans lequel le système comprend en outre une base de données pour stocker les résultats de traitement et les données du ou des processeurs, et le ou les processeurs peuvent récupérer la base de données.

11. Système selon la revendication 8, dans lequel le ou les processeurs peuvent être commandés à distance, et peuvent être utilisés pour observer à distance le fonctionnement du système en mode temps réel.

12. Système selon l'une quelconque des revendications 8 à 11, dans lequel le terminal comprend en outre une interface homme-machine pour commander le système et/ou afficher les résultats.

FIG. 1

FIG. 2

FIG. 3

FIG. 4

Generating multiple synchronous orthogonal sine waves from frequency domain to time domain ——— S511

Converting the digital signals into analog voltage signals ——— S512

Amplifying the analog signals to drive the current pump ——— S513

Converting the voltage signals into current signals ——— S514

Injecting the multiple synchronous orthogonal sine wave currents into the human or animal body ——— S515

FIG. 5a

Receiving the analog voltage signals from the human or animal body ——— S521

Amplifying the analog voltage signals ——— S522

Converting the analog signals into digital signals ——— S523

FIG. 5b

| Performing Fourier Transform to convert the signals from the time domain to the frequency domain in order to obtain wideband frequency responses | S531 |

| Preparing time sequences of the transmitted multi-frequency signals' amplitude and phase | S532 |

| Filtering every sequence | S533 |

| Obtaining a time sequence of the frequency responses | S534 |

| Calculating the system transfer function at each time point, to obtain a time sequence of the system transfer function | S535 |

| Calculating the heart's resistance and capacitance from the system transfer function at each time point, based on the multi-chamber model | S536 |

| Obtaining the time sequences of the heart's resistance and capacitance | S537 |

FIG. 5c

S531 ......in order to obtain amplitudes and phases
S532 ......amplitudes and phases
S536 ...... each time point by using the multi-chamber model

| Removing the geometric information, and converting the time sequence of the heart's capacitance to a motion sequence of the myocardial cells | S541 |

| Analyzing the motion sequence of the myocardial cells for the myocardial diseases | S542 |

FIG. 5d

S542 Analyzing the motion sequence of the myocardial cells and the myocardial disease

FIG. 6a

FIG. 6b

FIG. 6c

FIG. 6d

FIG. 7a

FIG. 7b

FIG. 7c

FIG. 7d

FIG. 8a

FIG. 8b

FIG. 8c

FIG. 8d

FIG. 9a

FIG. 9b

FIG. 9c

FIG. 9d

FIG. 10a

FIG. 10b

FIG. 10c

FIG. 10d

FIG. 11a

FIG. 11b

FIG. 11c

FIG. 11d

FIG. 12a

FIG. 12b

FIG. 12c

FIG. 12d

FIG. 13a

FIG. 13b

FIG. 13c

FIG. 13d

FIG. 14a

FIG. 14b

FIG. 14c

FIG. 14d

FIG. 15a

FIG. 15b

FIG. 15c

dc/dt/△C ( $\dot{\varepsilon}$=0.41)

FIG. 15d

ECG

FIG. 16a

Rh

FIG. 16b

Ch (ρ=0%)

FIG. 16c

dc/dt/△C ( $\dot{\varepsilon}$=-0.62)

FIG. 16d

ECG

FIG. 17a

FIG. 17b

FIG. 17c

FIG. 17d

FIG. 18a

FIG. 18b

FIG. 18c

FIG. 18d

FIG. 19a

FIG. 19b

FIG. 19c

FIG. 19d

FIG. 20a

FIG. 20b

FIG. 20c

FIG. 20d

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9853737 A1

- EP 3957240 A1